# EUROPEAN PATENT APPLICATION

(11) **EP 3 223 018 A1**
(43) Date of publication of application: **27.09.2017**
(21) Application number: 16162173.5
(22) Date of filing: 24.03.2016
(51) Int. Cl.: G01N 33/72

(54) **SYSTEM FOR MEASURING TOTAL HEMOGLOBIN IN BLOOD AND METHOD OF DOING THE SAME**

(71) Applicant: Mpock, Emmanuel C., Salida, CA 95368 (US)
(72) Inventor: Mpock, Emmanuel C., Salida, CA 95368 (US)
(74) Representative: Docherty, Robert Charles

(57) **Abstract**

A system can be used for determining a total hemoglobin concentration in a blood sample. The system has a strip that has a capillary channel having an entrance and a vent. A disposable body is housed within the capillary channel and adapted to receive a flow of blood from the entrance such that air in the disposable body is pushed out through a vent. A measurement system is configured to measure a property of the flow of blood. A non-transitory computer readable medium stores a program in memory that causes a processor to execute a process for determining the total hemoglobin concentration in the blood sample. The process involves receiving a first measurement from the measurement system. Then, calculating the total hemoglobin concentration in the blood sample based on the property. Finally, displaying the total hemoglobin concentration in the blood sample.

## Description

### BACKGROUND

The embodiments described herein relate generally to the measurement of hemoglobin in the blood, and more particularly, to methods and corresponding systems for measuring the total hemoglobin in whole blood using its physical, optical and electrical properties.

Conventionally, measuring the total amount of hemoglobin in blood is done indirectly by lysing the red blood cells exposing released hemoglobin molecules to other molecules to which they bind to form stable compounds with characteristics that allow them to be optically or electrochemically measured. The resulting concentrations of these compounds are typically directly proportional to the amount of hemoglobin in the sample. However, chemical interference from medications, other blood components, and incomplete reagent rehydration can impact the accuracy and prolong the reaction time required for known methods.

Therefore, what is needed is a rapid system and method for the direct quantification of total hemoglobin in a system designed for use in any setting, wherein a very small amount of whole blood sample is required.

### Statement of Invention

A system can be used for determining a total hemoglobin concentration in a blood sample. The system has a strip that has a capillary channel having an entrance and a vent. A disposable body is housed within the capillary channel and adapted to receive a flow of blood from the entrance such that air in the disposable body is pushed out through a vent. A measurement system is configured to measure a property of the flow of blood. A non-transitory computer readable medium stores a program in memory that causes a processor to execute a process for determining the total hemoglobin concentration in the blood sample. The process involves receiving a first measurement from the measurement system. Then, calculating the total hemoglobin concentration in the blood sample based on the property. Finally, displaying the total hemoglobin concentration in the blood sample.

### BRIEF DESCRIPTION OF THE FIGURES

The detailed description of some embodiments of the invention is made below with reference to the accompanying figures, wherein like numerals represent corresponding parts of the figures.
Figure 1 shows a schematic of an embodiment of the solid disposable unitary body configured for optical measurements;
Figure 2 shows a schematic of an embodiment of a solid disposable unitary body configured for electrical measurements;
Figure 3 shows a graph of a flow to static delta versus reference total hemoglobin values (data from physical measurement);
Figure 4 shows a schematic of an embodiment of the system;
Figure 5 is a graph of the total hemoglobin versus reciprocal of the broad blue signal (data from spectral measurement);
Figure 6 is a graph of the total hemoglobin versus multiple wavelength ratio like blue and red (data from spectral measurement);
Figure 7 is a graph of the total hemoglobin versus flow speed (data from physical measurement);
Figure 8 is a graph of the total hemoglobin versus impedance (data from electrical measurement);
Figure 9 is a schematic of an embodiment of a circuit for impedance or resistance measurement;
Figure 10 is a schematic of an embodiment of a strip.

### DETAILED DESCRIPTION OF CERTAIN EMBODIMENTS

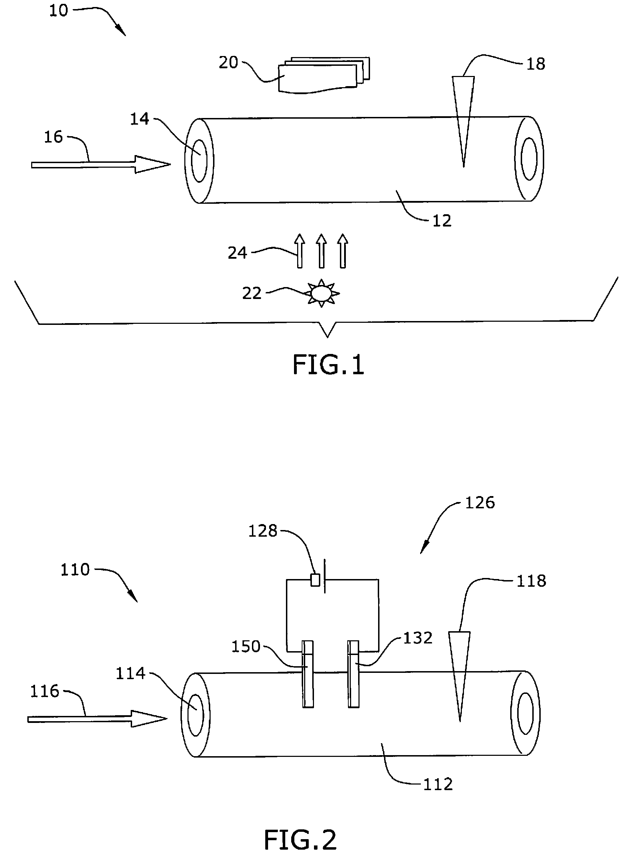

By way of example, and referring to Figure 1, one embodiment of measurement system 10 comprises disposable body 12. Disposable body 12 further comprises central channel 14. Central channel 14 is adapted to receive a flow of blood 16. Flow of blood 16 will pass through central channel 14 unless valve 18 in central channel 14 is closed. Diode for optical detection 20 is at a distal end of disposable body 12. Radiation emitter 22 produces radiation 24 which travels through disposable body 12, channel 14, blood 16 and into diode for optical detection 20.

Turning to Figure 2, one embodiment of measurement system 110 comprises disposable body 112. Disposable body 112 further comprises central channel 114. Central channel 114 is adapted to receive a flow of blood 116. Flow of blood 116 will pass through central channel 114 unless valve 118 in central channel 114 is closed. Measurement circuit 126 further comprises power source 128 connected to first lead 130 and second lead 132. Measurement circuit 126 measures either resistance (if using direct current) or impedance (if using alternating circuit) to determine the total hemoglobin level of blood 116.

The disposable body may have any desired size and, in some embodiments, comprises a channel having a height of from about 0.002 to about 0.050 inches, a length of from about 0.25 to about 2.0 inches, and a width of from about 0.02 to about 0.2 inches. The channel may hold a sample volume ranging from about 1 to about 10 microliters. The channel may have a predesigned size adapted for making photometric or electrical measurements with undiluted whole blood or blood derivatives. For example, the body and channel may be cylindrical or a slide and may comprise a plastic or metal plastic hybrid.

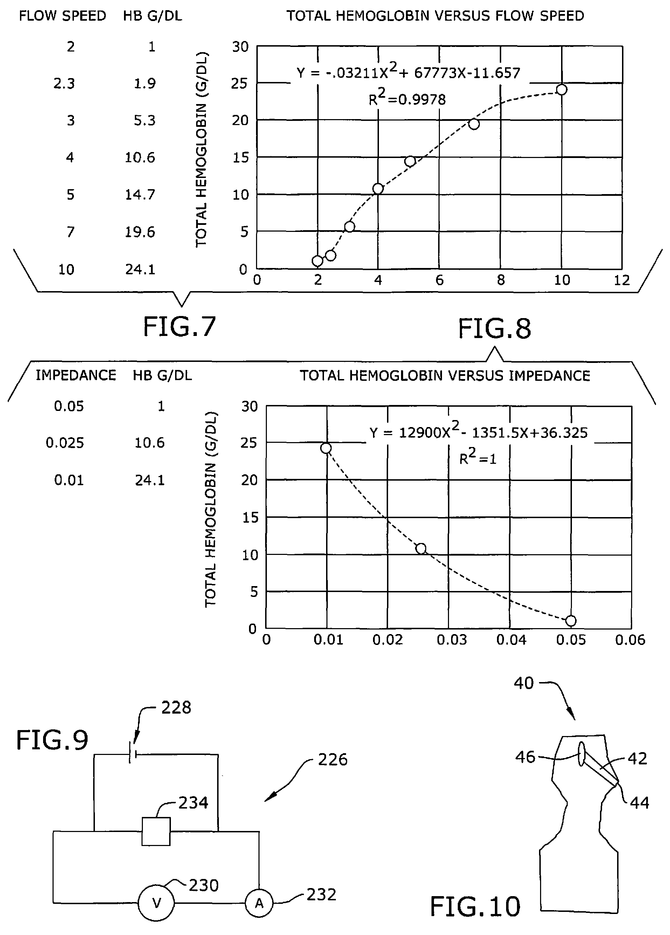

Turning to Figure 9, measurement circuit 226 further comprises power source 228 in parallel with voltmeter 230, ammeter 232 and load 234. By using Ohm's law a user with knowledge of voltage and amperage will be able to determine resistance or impedance.

Turning to Figure 10, measurement system 10 can be embodied into strip 40. Strip 40 further comprises capillary channel 42 which can house disposable body 12. Capillary channel 42 further comprises entrance 44 and vent 46. This enables flow of blood 16 to push air from capillary channel 42 and out of vent 46.

Strip 40 must be made such that surface energy of material is the same on both sides of the strip. This controls movement of blood 16 and ensures that movement of blood 16 is accurate for measurement. For instance, a sufficiently hydrophilic material is needed such as glass. A hydrophobic material will result in blood 16 beading and causing errors in the reading. Whether a compound is sufficiently hydrophilic can be determined by measuring the number of Hydroxyl groups (-OH), found in alcohols which are polar and therefore hydrophilic.

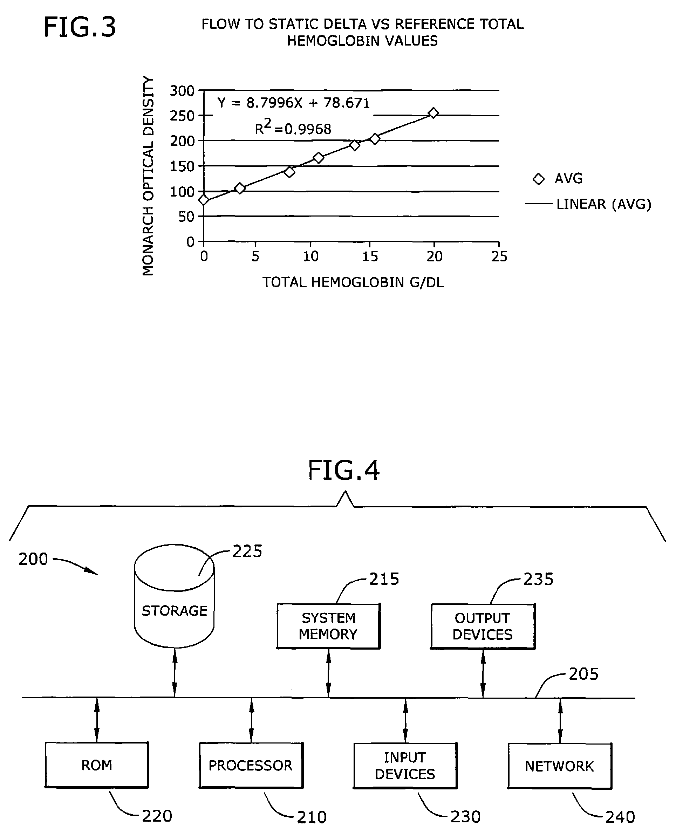

Turning first to light based testing, a control light needs to be selected by the user. In general, white light will be slightly distorted if there is excessive amounts of fat in blood, but the wavelength could be broken up into red, orange, yellow, green, blue, indigo and violet. An example using blue light is shown in Figure 3. To complete this experiment light is first reflected from radiation emitter 22 into optical detection diode 24 to obtain a control optical density (or absorbance). After that, blood 16 is introduced into the system and the same light is passed through the blood. The absorbance is the natural log of the radiant flux transmitted divided by the radiant flux received. This is graphed for blue light to determine total hemoglobin in Figure 3. Approximately, 3-4 microliters of blood are required to complete this test, which is much less than the 10 microliters currently required in testing.

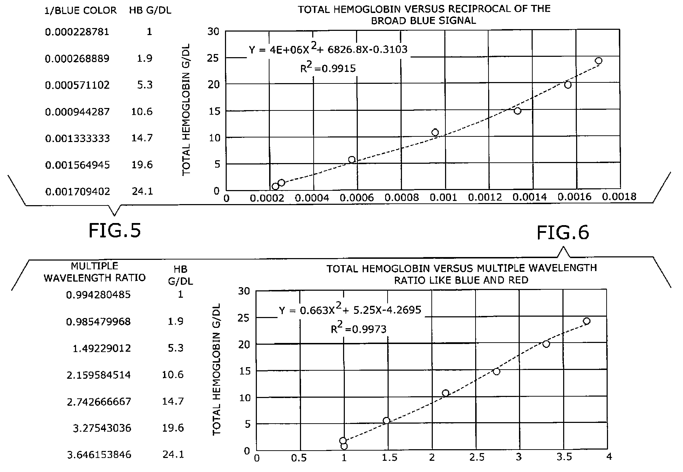

Turning to Figure 5 and Figure 6 accuracy can be increased by breaking the white light into multiple wavelengths and then averaging the results to determine a closer correlation of the variables. Turning to Figure 7, Flow speed of the blood filling the capillary can be measured to determine total hemoglobin level. As shown in Figure 8, impedence across electrodes can be used to determine total hemoglobin. In some cases this only requires 1.5-2 microliters of blood.

Figure 4 conceptually illustrates an electronic system 200 with which some embodiments of the invention are implemented. The electronic system 200 may be a computer, phone, PDA, or any other sort of electronic device. Such an electronic system includes various types of computer readable media and interfaces for various other types of computer readable media. Electronic system 200 includes a bus 205, processing unit(s) 210, a system memory 215, a read-only 220, a permanent storage device 225, input devices 230, output devices 235, and a network 240.

The bus 205 collectively represents all system, peripheral, and chipset buses that communicatively connect the numerous internal devices of the electronic system 200. For instance, the bus 205 communicatively connects the processing unit(s) 210 with the read-only 220, the system memory 215, and the permanent storage device 225.

From these various memory units, the processing unit(s) 210 retrieves instructions to execute and data to process in order to execute the processes of the invention. The processing unit(s) may be a single processor or a multi-core processor in different embodiments.

The read-only-memory (ROM) 220 stores static data and instructions that are needed by the processing unit(s) 210 and other modules of the electronic system. The permanent storage device 225, on the other hand, is a read-and-write memory device. This device is a nonvolatile memory unit that stores instructions and data even when the electronic system 200 is off. Some embodiments of the invention use a mass-storage device (such as a magnetic or optical disk and its corresponding disk drive) as the permanent storage device 225.

Other embodiments use a removable storage device (such as a floppy disk or a flash drive) as the permanent storage device 225. Like the permanent storage device 225, the system memory 215 is a read-and-write memory device. However, unlike storage device 225, the system memory 215 is a volatile read-and-write memory, such as a random access memory. The system memory 215 stores some of the instructions and data that the processor needs at runtime. In some embodiments, the invention's processes are stored in the system memory 215, the permanent storage device 225, and/or the read-only 220. For example, the various memory units include instructions for processing appearance alterations of displayable characters in accordance with some embodiments. From these various memory units, the processing unit(s) 210 retrieves instructions to execute and data to process in order to execute the processes of some embodiments.

The bus 205 also connects to the input and output devices 230 and 235. The input devices enable the person to communicate information and select commands to the electronic system. The input devices 230 include alphanumeric keyboards and pointing devices (also called "cursor control devices"). The output devices 235 display images generated by the electronic system 200. The output devices 235 include printers and display devices, such as cathode ray tubes (CRT) or liquid crystal displays (LCD). Some embodiments include devices such as a touchscreen that functions as both input and output devices.

Finally, as shown in Figure 4, bus 205 also couples electronic system 200 to a network 240 through a network adapter (not shown). In this manner, the computer can be a part of a network of computers (such as a local area network ("LAN"), a wide area network ("WAN"), or an intranet), or a network of networks (such as the Internet). Any or all components of electronic system 200 may be used in conjunction with the invention.

These functions described above can be implemented in digital electronic circuitry, in computer software, firmware or hardware. The techniques can be implemented using one or more computer program products. Programmable processors and computers can be packaged or included in mobile devices. The processes may be performed by one or more programmable processors and by one or more set of programmable logic circuitry. General and special purpose computing and storage devices can be interconnected through communication networks.

Some embodiments include electronic components, such as microprocessors, storage and memory that store computer program instructions in a machine-readable or computer-readable medium (alternatively referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray® discs, ultra density optical discs, any other optical or magnetic media, and floppy disks. The computer-readable media may store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

As used in this application, the term "a" or "an" means "at least one" or "one or more."

As used in this application, the term "about" or "approximately" refers to a range of values within plus or minus 10% of the specified number.

As used in this application, the term "substantially" means that the actual value is within about 10% of the actual desired value, particularly within about 5% of the actual desired value and especially within about 1% of the actual desired value of any variable, element or limit set forth herein.

All references throughout this application, for example patent documents including issued or granted patents or equivalents, patent application publications, and non-patent literature documents or other source material, are hereby incorporated by reference herein in their entireties, as though individually incorporated by reference, to the extent each reference is at least partially not inconsistent with the disclosure in the present application (for example, a reference that is partially inconsistent is incorporated by reference except for the partially inconsistent portion of the reference).

A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

Any element in a claim that does not explicitly state "means for" performing a specified function, or "step for" performing a specified function, is not to be interpreted as a "means" or "step" clause as specified in 35 U.S.C. § 112, ¶ 6. In particular, any use of "step of" in the claims is not intended to invoke the provision of 35 U.S.C. §112, ¶ 6.

Persons of ordinary skill in the art may appreciate that numerous design configurations may be possible to enjoy the functional benefits of the inventive systems. Thus, given the wide variety of configurations and arrangements of embodiments of the present invention the scope of the invention is reflected by the breadth of the claims below rather than narrowed by the embodiments described above.

## Claims

1. A system for determining a total hemoglobin concentration in a blood sample; the system comprising:
a strip further comprising: a capillary channel having an entrance and a vent;
a disposable body, housing the capillary channel and adapted to receive a flow of blood from the entrance such that air in the disposable body is pushed out through a vent;
a measurement system configured to measure a property of the flow of blood;
a non-transitory computer readable medium, storing a program in memory that causes a processor to execute a process for determining the total hemoglobin concentration in the blood sample, the process comprising:
receiving a first measurement from the measurement system;
calculating the total hemoglobin concentration in the blood sample based on the property;
displaying the total hemoglobin concentration in the blood sample.

2. The system of Claim 1, further comprising: diode for optical detection connected to the measurement system; and a radiation emitter which produces radiation that travels through the disposable body, the channel, the blood and into the diode for optical detection; wherein the first measurement is optical density of the-blood in motion.

3. The system of Claim 2, wherein the optical density is correlated to the total hemoglobin count with the equation Y = 8.7996X + 78.671.

4. The system of Claim 2 or 3, wherein the optical density is measured and correlated to the total hemoglobin for a static blood sample when the surface energy of the inner surface of a rectangular capillary system has a constant polarity.

5. The system of any one of Claims 1 to 4, further comprising: a measurement circuit, connected to the measurement system, and further comprising a power source connected to a first lead and a second lead; wherein the first measurement is impedance of the blood in motion.

6. The system of Claim 5, wherein the impedance is correlated to the total hemoglobin count with the equation Y = 12900X^2 - 1351.5X+36.325.

7. The system of Claim 5 or 6, wherein the impedance is measured and correlated to the total hemoglobin for a static blood sample when the surface energy of the inner surface of a rectangular capillary system has a constant polarity.

8. The system of Claim 7, wherein a height of the capillaries is less than 0.010 inches.
